(19) Europäisches Patentamt
European Patent Office
Office européen des brevets

(11) Publication number: 0 334 264 A2

(12) # EUROPEAN PATENT APPLICATION

(21) Application number: 89104963.7

(22) Date of filing: 20.03.89

(51) Int. Cl.[4]: A61K 37/64 , A61K 31/44 , A61K 45/06

(30) Priority: 21.03.88 US 171068

(43) Date of publication of application: 27.09.89 Bulletin 89/39

(84) Designated Contracting States: DE FR GB IT

(71) Applicant: E.R. Squibb & Sons, Inc.
Lawrenceville-Princeton Road
Princeton, N.J. 08540(US)

(72) Inventor: Sudilovsky, Abraham
712 Winchester Avenue
Lawrenceville New Jersey(US)

(74) Representative: Vossius & Partner
Siebertstrasse 4 P.O. Box 86 07 67
D-8000 München 86(DE)

(54) **Pharmaceutical compositions for treating tardive dyskinesia employing an ace inhibitor.**

(57) A pharmaceutical composition is provided for treating or inhibiting onset of tardive dyskinesia in a mammalian species containing an ACE inhibitor, such as captopril, alone or in combination with a calcium channel blocker such as diltiazem, nifedipine or verapamil.

EP 0 334 264 A2

# PHARMACEUTICAL COMPOSITIONS FOR TREATING TARDIVE DYSKINESIA EMPLOYING AN ACE INHIBITOR

The present invention relates to pharmaceutical compositions for preventing or treating tardive dyskinesia in mammalian species by using an ACE inhibitor, such as captopril, zofenopril or fosinopril alone or in combination with a calcium channel blocker.

In accordance with the present invention, a pharmaceutical composition is provided for preventing or treating tardive dyskinesia in mammalian species containing an angiotensin converting enzyme inhibitor in an amount effective to prevent or treat tardive dyskinesia. It is administered systemically, such as orally or parenterally.

The angiotensin converting enzyme inhibitor may be administered alone or in combination with a calcium channel blocker.

Where a combination of ACE inhibitor and calcium channel blocker are to be used, the ACE inhibitor will be employed in a weight ratio to the calcium channel blocker of within the range of from about 0.1:1 to about 10:1 and preferably from about 0.4:1 to about 2.5:1.

The angiotensin converting enzyme inhibitor which may be employed herein includes substituted proline derivatives, such as any of those disclosed in U. S. Patent No. 4,046,889 to Ondetti et al mentioned above, with captopril, that is, 1-[(2S)-3-mercapto-2-methylpropionyl]-L-proline, being preferred, carboxyalkyl dipeptide derivatives, such as any of those disclosed in European Patent Application 0 012 401 mentioned above, with N-(1-ethoxycarbonyl-3-phenylpropyl)-L-alanyl-L-proline, that is, enalapril, being preferred.

Other examples of angiotensin converting enzyme inhibitors suitable for use herein include any of the phosphonate substituted amino or imino acids or salts disclosed in U. S. Patent No. 4,452,790 with (S)-1-[6-amino-2-[[hydroxy-(4-phenylbutyl)phosphinyl]oxy]-1-oxohexyl]-L-proline being preferred, phosphinylalkanoyl prolines disclosed in U. S. Patent No. 4,168,267 mentioned above with fosinopril being preferred, mercaptoacyl derivatives of substituted prolines disclosed in U. S. Patent No. 4,316,906 with zofenopril being preferred, any of the phosphinylalkanoyl substituted prolines disclosed in U. S. Patent No. 4,337,201 discussed above, and the phosphonamidates disclosed in U. S. Patent No. 4,432,971 discussed above.

Other examples of ACE inhibitors that may be employed herein include Beecham's BRL 36,378 as disclosed in European patent Nos. 80822 and 60668; Chugai's MC-838 disclosed in CA. 102:72588v and Jap. J. Pharmacol. 40:373 (1986); Ciba-Geigy's CGS 14824 (3-([1-ethoxycarbonyl-3-phenyl-(1S)-propyl]-amino)-2,3,4,5-tetrahydro-2-oxo-1-(3S)-benzazepine-1 acetic acid HCl) disclosed in U.K. Patent No. 2103614 and CGS 16,617 (3(S)-[[(1S)-5-amino-1-carboxypentyl]amino]-2,3,4,6-tetrahydro-2-oxy-1H-1-benzazepine-1-ethanoic acid) disclosed in U. S. Patent No. 4,473,575; cetapril (alacepril, Dainippon) disclosed in Eur. Therap. Res. 39:671 (1986); 40:543 (1986); ramipril (Hoechst) disclosed in Eur. Patent No. 79-022 and Curr. Ther. Res. 40:74 (1986); Ru 44570 (Hoechst) disclosed in Arzneimittelforschung 35:1254 (1985), cilazapril (Hoffman-LaRoche) disclosed in J. Cardiovasc. Pharmacol. 9:39 (1987); $R_o$ 31-2201 (Hoffman-LaRoche) disclosed in FEBS Lett. 165:201 (1984); lisinopril (Merck) disclosed in Curr. Therap. Res.37:342 (1985) and Eur. patent appl. No. 12-401, indalapril (delapril) disclosed in U. S. Patent No. 4,385,051; rentiapril (fentiapril, Santen) disclosed in Clin. Exp. Pharmacol. Physiol. 10:131 (1983); indolapril (Schering) disclosed in J. Cardiovasc. Pharmacol. 5:643, 655 (1983); spirapril (Schering) disclosed in Acta. Pharmacol. Toxicol. 59 (Supp. 5):173 (1986); perindopril (Servier) disclosed in Eur. J. Clin. Pharmacol. 31:519 (1987); quinapril (Warner-Lambert) disclosed in U. S. Patent No. 4,344,949 and CI 925 (Warner-Lambert) ([3S-[2[R(*)R(*)]]3R-(*)]-2-[2-[[1-(ethoxycarbonyl)-3-phenylpropyl]amino[-1-oxopropyl]-1,2,3,4-tetrahydro-6,7-dimethoxy-3-isoquinolinecarboxylic acid HCl) disclosed in Pharmacologist 26:243, 266 (1984), WY-44221 (Wyeth) disclosed in J. Med. Chem. 26:394 (1983).

The disclosure of the above-mentioned patents and other references are incorporated herein by reference.

The calcium antagonist which will be used herein may be diltiazem which is disclosed in U. S. Patent No. 3,562,257 and which has the chemical name 3-(acetyloxy)-5-[2-(dimethylamino)ethyl-2,3-dihydro-2-(4-methoxyphenyl)-1,5-benzothiazepin-4(5H)-one and the structure

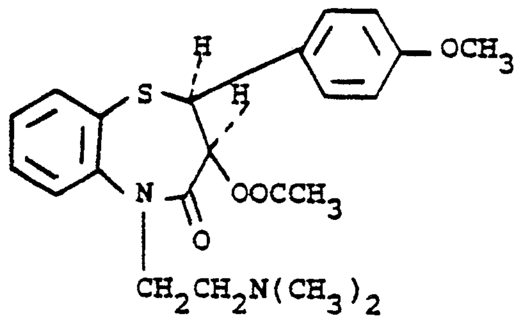

.HCl

Verapamil will also be a preferred calcium channel blocker.

4-Phenyl-1,4-dihydropyridine calcium antagonists may be employed which will have the structure

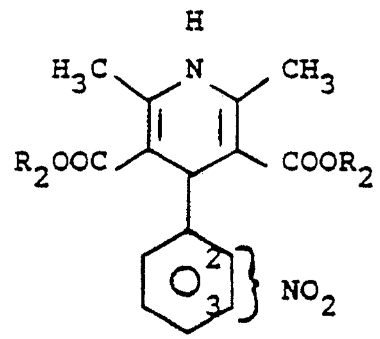

wherein $R_1$ and $R_2$ may be the same or different and are lower alkyl or lower alkoxy (lower alkyl) where lower alkyl and lower alkoxy contain 1 to 4 carbons.

The above compounds and methods for preparing same are disclosed in U. S. Patents Nos. 3,644,627, 3,485,847, 3,488,359, 3,574,843, 3,799,934, 3,932,645 and 4,154,839 which are incorporated herein by reference.

The dihydropyridine calcium antagonist present in the composition of the invention will preferably be nifedipine, that is, the compound of fromula C wherein $R_1$ is $CH_3$, $R_2$ or $CH_3$ and $NO_2$ is at the 2-position, namely,

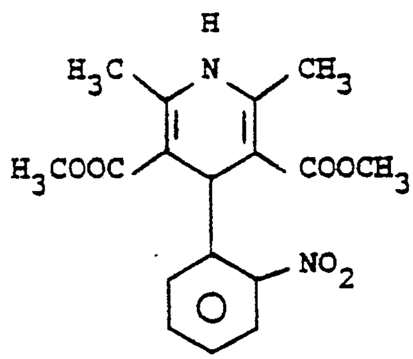

which is disclosed in U. S. Patents Nos. 3,644,627 and 3,485,847.

Other preferred 4-phenyl-1,4-dihydropyridine calcium antagonists suitable for use herein include niludipine, that is, the compound of formula C wherein $R_1$ is $-(CH_2)_2OC_3H_7$, $R_2$ is $-(CH_2)_2OC_3H_7$ and $NO_2$ is at the 3-position (disclosed in U. S. Patents Nos. 3,488,359 and 3,574,843); nimodipine, that is the compound of formula C wherein $R_1$ is $-(CH_2)_2OCH_3$, $R_2$ is $-CH(CH_3)_2$ and $NO_2$ is at the 3-position (disclosed in U. S. Patents Nos. 3,799,934 and 3,932,645); nitrendipine, that is, the compound of formula C wherein $R_1$ is $-CH_2CH_3$, $R_2$ is $-CH_3$ and $NO_2$ is at the 3-position (disclosed in U. S. Patents Nos. 3,799,934 and 3,932,645); and nisoldipine, that is, the compound of formula C wherein $R_1$ is $-CH_3$, $R_2$ is $-CH_2CH(CH_3)_2$ and $NO_2$ is at the 2-position (disclosed in U. S. Patents Nos. 3,799,934, 3,932,645 and 4,154,839),

that is, the compound of formula C wherein $R_1$ is $-CH_3$, $R_2$ is $-CH_2CH(CH_3)_2$ and $NO_2$ is at the 2-position (disclosed in U. S. Patents Nos. 3,799,934, 3,932,645 and 4,154,839). In carrying out the method of the invention for the preparation of pharmaceutical compositions, the angiotensin converting enzyme inhibitor alone or a combination of the angiotensin converting enzyme inhibitor and calcium channel blocker may be incorporated in a single conventional dosage form or each compound may be incorporated into a separate conventional dosage form to be taken at the same time. The dosage forms may comprise conventional oral forms, rectal forms or parenteral forms, such as tablets, capsules, suppositories, powders, ampoules, elixirs, suspensions, solutions, syrups, sustained release preparations and fluid injectable forms, such as sterile solutions. Oral dosage forms are preferred, although parenteral forms, such as intramuscular, intraperitoneal or intravenous forms are quite satisfactory as well.

The dose administered must be carefully adjusted according to age, weight and condition of the patient, as well as the route of administration, dosage form and regimen and the desired result.

Thus, for oral administration, a satisfactory result may be obtained employing the ACE inhibitor in an amount within the range of from about 0.01 mg/kg to about 100 mg/kg and preferably from about 0.1 mg/kg to about 25 mg/kg alone or in combination with the calcium channel blocker in an amount within the range of from about 0.01 mg/kg to about 100 mg/kg and preferably from about 0.1 mg/kg to about 25 mg/kg with the ACE inhibitor and calcium channel blocker being employed together in the same oral dosage form or in separate oral dosage forms taken at the same time.

A preferred oral dosage form , such as tablets or capsules, will contain the ACE inhibitor in an amount of from about 1 to about 500 mg, preferably from about 125 to about 200 mg, and more preferably from about 25 to about 150 mg alone or with the calcium channel blocker in an amount of from about 1 to about 350 mg, preferably from about 2 to about 200 mg, and more preferably from about 30 to about 150 mg.

For parenteral administration, the ACE inhibitor will be employed in an amount within the range of from about 0.005 mg/kg to about 10 mg/kg and preferably from about 0.01 mg/kg to about 1 mg/kg, alone or with the calcium channel blocker in an amount within the range of from about 0.005 mg/kg to about 20 mg/kg and preferbly from about 0.01 mg/kg to about 2 mg/kg.

The composition described above may be administered in the dosage forms as described above in single or divided doses of one to four times daily. It may be advisable to start a patient on a low dose combination and work up gradually to a high dose combination.

Tablets of various sizes can be prepared, e.g., of about 50 to 700 mg in total weight, containing one or both of the active substances in the ranges described above, with the remainder being a physiologically acceptable carrier of other materials according to accepted pharmaceutical practice. These tablets can, of course, be scored to provide for fractional doses. Gelatin capsules can be similarly formulated.

Liquid formulations can also be prepared by dissolving or suspending one or the combination of active substances in a conventional liquid vehicle acceptable for pharmaceutical administration so as to provide the desired dosage in one to four teaspoonsful.

Such dosage forms can be administered to the patient on a regimen of one to four doses per day.

According to another modification, in order to more finely regulate the dosage schedule, the active substances may be administered separately in individual dosage units at the same time or carefully coordinated times. Since blood levels are built up and maintained by a regulated schedule of administration, the same result is achieved by the simultaneous presence of the two substances. The respective substances can be individually formulated in separate unit dosage forms in a manner similar to that described above.

Fixed combinations of ACE inhibitor and calcium channel blocker are more convenient and are preferred, especially in tablet or capsule form for oral administration.

In formulating the compositions, the active substances, in the amounts described above, are compounded according to accepted pharmaceutical practice with a physiologically acceptable vehicle, carrier, excipient, binder, preservative, stabilizer, flavor, etc., in the particular type of unit dosage form.

Illustrative of the adjuvants which may be incorporated in tablets are the following: a binder such as gum tragacanth, acacia, corn starch or gelatin; an excipient such as dicalcium phosphate or cellulose; a disintegrating agent such as corn starch, potato starch, alginic acid or the like; a lubricant such as stearic acid or magnesium stearate; a sweetening agent such as sucrose, lactose or saccharin; a flavoring agent such as orange, peppermint, oil of wintergreen or cherry. When the dosage unit form is a capsule, it may contain in addition to materials of the above type a liquid carrier such as a fatty oil. Various other materials may be present as coatings or to otherwise modify the physical form of the dosage unit. For instance, tablets or capsules may be coated with shellac, sugar or both. A syrup of elixir may contain the active compound, water, alcohol or the like as the carrier, glycerol as solubilizer, sucrose as sweetening agent, methyl and propyl parabens as preservatives, a dye and a flavoring such as cherry or orange.

Many of the active substances described above form commonly known, pharmaceutically acceptable salts such as alkali metal and other common basic salts or acid addition salts, etc. References to the base substances are therefore intended to include those common salts known to be substantially equivalent to the parent compound.

The following Examples represent preferred embodiments of the present invention.

## Example 1

A captopril formulation suitable for oral administration in inhibiting onset of or treating tardive dyskinesia is set out below.

1000 tablets each containing 100 mg of 1-[(2S)-3-mercapto-2-methylpropionyl]-L-proline were produced from the following ingredients.

| | |
|---|---|
| 1-[(2S)-3-Mercapto-2-methylpropionyl]-L-proline (captopril) | 100 g |
| Corn starch | 50 g |
| Gelatin | 7.5 g |
| Avicel (microcrystalline cellulose) | 25 g |
| Magnesium stearate | 2.5 g |

The captopril and corn starch are admixed with an aqueous solution of the gelatin. The mixture is dried and ground to a fine powder. The Avicel and then the magnesium stearate are admixed with the granulation. This is then compressed in a tablet to form 1000 tablets each containing 100 mg of active ingredient which is used for inhibiting onset of or treating tardive dyskinesia.

## Example 2

By substituting 100 g of 1-(3-mercapto-2-D-methylpropanoyl)-L-proline for the captopril in Example 1, 1000 tablets each containing 100 mg of the 1-(3-mercapto-2-D-methylpropanoyl)-L-proline are produced which is useful in inhibiting onset of or treating tardive dyskinesia.

## Example 3

1000 tablets each containing 200 mg of captopril are produced from the following ingredients:

| | |
|---|---|
| Captopril | 200 g |
| Lactose | 100 g |
| Avicel | 150 g |
| Corn starch | 50 g |
| Magnesium stearate | 5 g |

The captopril, lactose and Avicel are admixed, then blended with the corn starch. Magnesium stearate is added. The dry mixture is compressed in a tablet press to form 1000 505 mg tablets each containing 200 mg of active ingredient. The tablets are coated with a solution of Methocel E 15 (methyl cellulose) including as a color a lake containing yellow #6. The resulting tablets are useful in inhibiting onset of or treating tardive dyskinesia.

## Example 4

Two piece #1 gelatin capsules each containing 250 mg of captopril are filled with a mixture of the following ingredients:

| | |
|---|---|
| Captopril | 250 mg |
| Magnesium stearate | 7 mg |
| USP lactose | 193 mg. |

The resulting capsules are useful in inhibiting onset of or treating tardive dyskinesia.

Example 5

An injectable solution for use in inhibiting onset of or treating tardive dyskinesia is produced as follows:

| | |
|---|---|
| Captopril | 500 mg |
| Methyl paraben | 5 mg |
| Propyl paraben | 1 mg |
| Sodium chloride | 25 g |
| Water for injection qs. | 5 l. |

The captopril, preservatives and sodium chloride are dissolved in 3 liters of water for injection and then the volume is brought up to 5 liters. The solution is filtered through a sterile filter and aseptically filled into presterilized vials which are then closed with presterilized rubber closures. Each vial contains 5 ml of solution in a concentration of 100 mg of active ingredient per ml of solution for injection.

Example 6

Tablets for use in inhibiting onset of or treating tardive dyskinesia are prepared as described in Example 1 except that N-(1-ethoxycarbonyl-3-phenylpropyl)-L-alanyl-L-proline (enalapril) is used in place of captopril.

Example 7

An injectable for use in inhibiting onset of or treating tardive dyskinesia is prepared as described in Example 5 except that N-(1-ethoxycarbonyl-3-phenylpropyl)-L-alanyl-L-proline (enalapril) is employed in place of captopril.

Example 8

A zofenopril formulation suitable for oral administration in inhibiting onset of or treating tardive dyskinesia is set out below.

1000 tablets each containing 100 mg of zofenopril are produced from the following ingredients.

| | |
|---|---|
| [1(S),4(S)]-1-[3-(benzoylthio))-2-methyl-1-oxopropyl-4-(phenylthio)-L-proline (zofenopril) | 100 g |
| Corn starch | 50 g |
| Gelatin | 7.5 g |
| Avicel (microcrystalline cellulose) | 25 g |
| Magnesium stearate | 2.5 g |

The zofenopril and corn starch are admixed with an aqueous solution of the gelatin. The mixture is dried and ground to a fine powder. The Avicel and then the magnesium stearate are admixed with the granulation. This is then compressed in a tablet to form 1000 tablets each containing 100 mg of active ingredient which is used for inhibiting onset of or treating tardive dyskinesia.

## Example 9

By substituting 100 g of fosinopril for the zofenopril in Example 8, 1000 tablets each containing 100 mg of the fosinopril are produced which is useful in inhibiting onset of or treating tardive dyskinesia.

## Example 10

1000 tablets each containing 200 mg of fosinopril are produced from the following ingredients:

| | |
|---|---|
| Fosinopril | 200 g |
| Lactose | 100 g |
| Avicel | 150 g |
| Corn starch | 50 g |
| Magnesium stearate | 5 g |

The fosinopril, lactose and Avicel are admixed, then blended with the corn starch. Magnesium stearate is added. The dry mixture is compressed in a tablet press to form 1000 505 mg tablets each containing 200 mg of active ingredient. The tablets are coated with a solution of Methocel E 15 (methyl cellulose) including as a color a lake containing yellow #6. The resulting tablets are useful in onset of or treating tardive dyskinesia.

## Example 11

Tablets for use in inhibiting onset of or treating tardive dyskinesiaare prepared as described in Example 1 except that 1-[N-[hydroxy- (4-phenylbutyl)phosphinyl]-L-alanyl-L-proline, disodium salt (prepared as described in U. S. Patent No. 4,432,971) is used in place of captopril.

## Example 12

A captopril-diltiazem formulation suitable for oral administration in the treatment of tardive dyskinesia is set out below.

1000 tablets each containing 100 mg of 1-[(2S)-3-mercapto-2-methylpropionyl]-L-proline and 100 mg of diltiazem are produced from the following ingredients:

| | |
|---|---|
| 1-(2S)-3-mercapto-2-methylpropionyl]-L-proline (captopril) | 100 g |
| Diltiazem | 100 g |
| Corn starch | 50 g |
| Gelatin | 7.5 g |
| Avicel (microcrystalline cellulose) | 25 g |
| Magnesium stearate | 2.5 g |

The captopril, diltiazem and corn starch are admixed with an aqueous solution of the gelatin. The mixture is dried and ground to a fine powder. The Avicel and then the magnesium stearate are admixed with the granulation. This is then compressed in a tablet to form 1000 tablets each containing 200 mg of active ingredients which is used for prevention or treating tardive dyskinesia.

## Example 13

By substituting 100 g of 1-(3-mercapto-2-D-methylpropanoyl)-L-proline for the captopril in Example 1, 1000 tablets each containing 100 mg of the 1-(3-mercapto-2-D-methylpropanoyl)-L-proline and 100 mg diltiazem are produced which is useful in preventing or treating tardive dyskinesia.

## Example 14

1000 tablets each containing 200 mg of captopril and 200 mg nifedipine are produced from the following ingredients:

| | |
|---|---|
| Captopril | 200 g |
| Nifedipine | 200 g |
| Lactose | 100 g |
| Avicel | 150 g |
| Corn starch | 50 g |
| Magnesium stearate | 5 g |

The captopril, diltiazem, lactose and Avicel are admixed, then blended with the corn starch. Magnesium stearate is added. The dry mixture is compressed in a tablet press to form 1000 505 mg tablets each containing 200 mg of each active ingredient. The tablets are coated with a solution of Methocel E 15 (methyl cellulose) including as a color a lake containing yellow #6. The resulting tablets are useful in preventing or treating tardive dyskinesia.

## Example 15

Two piece #1 gelatin capsules each containing 250 mg of enalapril and 150 mg of nitrendipine are filled with a mixture of the following ingredients:

| | |
|---|---|
| Enalapril | 250 mg |
| Nitrendipine | 150 mg |
| Magnesium stearate | 7 mg |
| USP lactose | 193 mg |

The resulting capsules are useful in preventing or treating tardive dyskinesia

## Example 16

An injectable solution for use in treating or preventing tardive dyskinesia is produced as follows:

| | |
|---|---|
| Captopril | 500 mg |
| Diltiazem | 300 mg |
| Methyl paraben | 5 g |
| Propyl paraben | 1 g |
| Sodium chloride | 25 g |
| Water for injection qs. | 5 L. |

The captopril, diltiazem, preservatives and sodium chloride are dissolved in 3 liters of water for injection and then the volume is brought up to 5 liters. The solution is filtered through a sterile filter and aseptically filled into presterilized vials which are then closed with presterilized rubber closures. Each vial contains 5 ml of solution in a concentration of 100 mg of active ingredient per ml of solution for injection.

## Example 17

Tablets for use in treating tardive dyskinesia are prepared as described in Example 12 except that N-(1-ethoxycarbonyl-3-phenylpropyl)-L-alanyl-L-proline (enalapril) is used in place of captopril and nifedipine is used in place of diltiazem.

## Example 18

Tablets for use in treating tardive dyskinesia are prepared following the procedure of Example 12 except that zofenopril is employed in place of captopril and is used in place of diltiazem.

## Example 19

Tablets for use in treating tardive dyskinesia are prepared following the procedure of Example 12 except that fosinopril is employed in place of captopril.

## Example 20

Tablets for use in treating tardive dyskinesia are prepared following the procedure of Example 12 except that alacepril is employed in place of captopril.

## Example 21

Tablets for use in treating tardive dyskinesia are prepared following the procedure of Example 12 except that (S)-1-[6-amino-2-[[hydroxy-(4-phenylbutyl)phosphinyl]oxy]-1-oxohexyl]-L-proline or lisinopril is employed in place of captopril.

## Claims

1. Use of an angiotensin converting enzyme inhibitor alone or in combination with a calcium channel blocker for the preparation of a pharmaceutical composition for inhibiting onset of or treating tardive dyskinesia in a mammalian species.

2. Use as defined in Claim 1 wherein the angiotensin converting enzyme inhibitor is a phosphonate substituted amino or imino acid or salt thereof,, a substituted proline derivative, a carboxyalkyl dipeptide derivative, a phosphinylalkanoyl proline derivative or a phosphonamidate derivative.

3. Use as defined in Claim 1 wherein said angiotensin converting enzyme inhibitor alone or with a calcium channel blocker is administered orally or parenterally.

4. Use as defined in Claim 1 wherein said angiotensin converting enzyme inhibitor alone or with a calcium channel blocker is admixed with a pharmaceutically acceptable carrier therefor.

5. Use as defined in Claim 1 wherein said angiotensin converting enzyme inhibitor is a substituted proline derivative.

6. Use as defined in Claim 1 wherein said angiotensin converting enzyme inhibitor is a carboxyalkyl dipeptide derivative.

7. Use as defined in Claim 1 wherein said angiotensin converting enzyme inhibitor is a phosphinylalkanoyl proline derivative.

8. Use as defined in Claim 1 wherein said angiotensin converting enzyme inhibitor is a phosphonamidate derivative.

9. Use as defined in Claim 1 wherein said angiotensin converting enzyme inhibitor is a phosphonate substituted amino or imino acid or salt thereof.

10. Use as defined in Claim 1 wherein said angiotensin converting enzyme inhibitor is captopril.

11. Use as defined in Claim 1 wherein said angiotensin converting enzyme inhibitor is enalapril.

12. Use as defined in Claim 1 wherein said angiotensin converting enzyme inhibitor is 1-[N-[hydroxy(4-phenylbutyl)phosphinyl]-2-alanyl]-L-proline or its disodium salt.

13. Use as defined in Claim 1 wherein said angiotensin converting enzyme inhibitor is zofenopril.

14. Use as defined in Claim 1 wherein said angiotensin converting enzyme inhibitor is fosinopril.

15. Use as defined in Claim 1 wherein said angiotensin converting enzyme inhibitor is (S)-1-[6-amino-2-[[hydroxy(4-phenylbutyl)phosphinyl]oxy]-1-oxohexyl]-L-proline.

16. Use as defined in Claim 1 wherein the angiotensin converting enzyme inhibitor is administered with a calcium channel blocker.

17. Use as defined in Claim 16 wherein the calcium channel blocker is diltiazem, verapamil or a 4-phenyl-1,4-dihydropyridine.

18. Use as defined in Claim 17 wherein the 4-phenyl-1,4-dihydropyridine is nifedipine.

19. Use as defined in Claim 16 wherein the angiotensin converting enzyme inhibitor is employed in a weight ratio to the calcium channel blocker of within the range of from about 0.1:1 to about 10:1.

20. Use as defined in Claim 1 wherein said angiotensin converting enzyme inhibitor or a combination thereof with a calcium channel blocker is administered in the form of tablets, capsules or by injection.

21. Use as defined in Claim 1 wherein said angiotensin converting enzyme inhibitor in the pharmaceutical composition is captopril and is administered systemically in an amount of from about 0.1 to about 500 mg/one to four times a day.

22. Use as defined in Claim 1 wherein the pharmaceutical composition containing said angiotensin converting enzyme inhibitor is administered over a prolonged period of treatment.

23. Use as defined in Claim 1 wherein the angiotensin converting enzyme inhibitor is captopril, zofenopril, fosinopril, enalapril or (S)-1-[6-amino-2-[[hydroxy(4-phenylbutyl)phosphinyl]oxy]-1-oxohexyl-L-proline.